# EUROPÄISCHE PATENTSCHRIFT

(11) **EP 1 060 129 B1**
(45) Veröffentlichungstag und Bekanntmachung des Hinweises auf die Patenterteilung: **05.12.2001**
(21) Anmeldenummer: 99907537.7
(22) Anmeldetag: 11.02.1999
(51) Int. Cl.: C01G 51/00, C01G 53/00, C01G 39/00, B01J 23/88

(54) **MULTIMETALLOXIDMASSEN**
POLYMETALLIC OXIDE MATERIALS
PRODUITS D'OXYDES POLYMETALLIQUES

(30) Priorität: 20.02.1998 DE 19807269; 24.08.1998 DE 19838312
(43) Veröffentlichungstag der Anmeldung: 20.12.2000
(73) Patentinhaber: BASF AKTIENGESELLSCHAFT, 67056 Ludwigshafen (DE)
(72) Erfinder: JACHOW, Harald, D-64625 Bensheim (DE)
(86) Internationale Anmeldenummer: EP9900890
(87) Internationale Veröffentlichungsnummer: WO9942404

(56) Entgegenhaltungen:
- DE-A- 2 042 216
- KEELY: "preparation techniques for hydrotreating catalysts..." 8TH INTERNATIONAL CONGRESS ON CATALYSIS, Bd. IV, 1985, Seiten 403-414, XP002106522

## Beschreibung

Vorliegende Erfindung betrifft Multimetalloxidmassen der allgemeinen Formel I

Mₐ ¹Mo_{1-b}M_{b} ²Oₓ (I),

mit
- M¹ =: Co, Ni, Mg, Zn, Mn und/oder Cu, vorzugsweise Co, Ni und/oder Mg, besonders bevorzugt Co und/oder Ni,
- M² =: W, V, Te, Nb, P, Cr, Fe, Sb, Ce, Sn und/oder La, vorzugsweise Sn, W, P, Sb und/oder Cr, besonders bevorzugt W, Sn und/oder Sb,
- a =: 0,5 bis 1,5, vorzugsweise 0,7 bis 1,2, besonders bevorzugt 0,9 bis 1,0,
- b =: 0 bis 0,5, vorzugsweise > 0 bis 0,5 und besonders bevorzugt 0,01 bis 0,3 sowie
- x =: eine Zahl, die durch die Wertigkeit und Häufigkeit der von Sauerstoff verschiedenen Elemente in (I) bestimmt wird,
mit der Maßgabe, daß
der mittlere Durchmesser der Poren der Multimetalloxidmasse (1) ≤ 0,04 µm und ≥ 0,01 µm und
die spezifische Oberfläche der Multimetalloxidmasse (I) ≥ 20 m²/g beträgt.

Ferner betrifft vorliegende Erfindung die gasphasenkatalytisch oxidative Dehydrierung von Propan zu Propen, unter Verwendung der Multimetalloxidmassen (I) als Katalysatoren.

Multimetalloxidmassen der Stöchiometrie (I) sind bekannt. Beispielsweise werden derartige Multimetalloxidmassen in Topics in Catalysis 3 (1996) 265-275, in Catalysis Letters 35 (1995) 57-64, in Catalysis Today 24 (1995) 327-333, in Ind. Eng. Chem. Res. 1996, 35, 14-18, in US-A 4,255,284, in 3^{rd} world Congress on Oxidation Catalysis, R.K. Grasselli et. al.(Editors) 1997 Elsevier Science B.V., 357-364, in J. of Catalysis 167, 560-569 (1997), in Catalysis Letters 10 (1991) 181-192, in US-A 5,086,032, in US-A 4,255,284, in US-A 5,086,032, in J. of Catalysis 170, 346-356 (1997) und in der älteren Anmeldung DE-A 19751046 als geeignete Katalysatoren für die gasphasenkatalytische oxidative Dehydrierung von Propan zu Propen beschrieben. Nachteilig an den vorgenannten Multimetalloxidmassen des Standes der Technik ist jedoch, daß die mit ihnen bei vorgegebener Belastung mit Propan und molekularem Sauerstoff enthaltendem Reaktionsgasausgangsgemisch bei vorgegebenen Reaktionsbedingungen erzielbaren Raum-Zeit-Ausbeuten an Propen bzw. an Propen, Acrolein und Acrylsäure ( gemeinsam : Wertprodukt )nicht zu befriedigen vermögen.

Die Aufgabe der vorliegenden Erfindung bestand daher darin, neue Multimetalloxidmassen zur Verfügung zu stellen, die bei Verwendung als Katalysatoren für die katalytische oxidative Dehydrierung von Propan zu Propen bzw. zu Propen, Acrolein und Acrylsäure bei vorgegebener Belastung und vorgegebenen Reaktionsbedingungen erhöhte Raum-Zeit-Ausbeuten ermöglichen.

Demgemäß wurden die eingangs definierten Multimetalloxidmassen (I) gefunden.

Erfindungsgemäß bevorzugte Multimetalloxidmassen (I) sind solche der allgemeinen Formel (II)

[Co, Ni u./o. Mg]ₐ Mo_{1-b} [Sn, W, P, Sb u./o. Cr]_{b} Oₓ (II),

mit
- a =: 0,5 bis 1,5, vorzugsweise 0,7 bis 1,2, besonders bevorzugt 0,9 bis 1.0.
- b =: 0 bis 0,5, vorzugsweise > 0 bis 0,5 und besonders bevorzugt 0,01 bis 0,3 sowie
- x =: eine Zahl, die durch die Wertigkeit und Häufigkeit der von Sauerstoff verschiedenen Elemente in (II) bestimmt wird.

Erfindungsgemäß besonders bevorzugte Multimetalloxidmassen (I) sind solche der allgemeinen Formel (III)

[Co u./o. Ni]ₐ Mo_{1-b} [W, Sn u./o. Sb]_{b} Oₓ (III),

mit den vorstehenden Bedeutungen für a, b und x.

Ferner sind solche Multimetalloxidmassen (I), (II) und (III) bevorzugt, deren mittlerer Durchmesser ihrer Poren ≥ 0,02 µm und ≤ 0,035 µm beträgt. Darüber hinaus eignen sich erfindungsgemäß solche Multimetalloxidmassen (I), (II) und (III), deren mittlerer Durchmesser ihrer Poren ≥ 0,025 µm und ≤ 0,030 µm beträgt.

Ferner ist es günstig, wenn die vorgenannten Multimetalloxidmassen bei vorgenannten mittleren Porendurchmessern gleichzeitig eine spezifische Oberfläche von ≥ 25 m²/g bzw. von ≥ 30m²/g aufweisen. In der Regel wird die spezifische Oberfläche der erfindungsgemäßen Multimetalloxidmassen ≤ 50 m²/g betragen.

Unter der spezifischen Oberfläche O wird in dieser Schrift die gemäß DIN 66133 mittels der Methode der Quecksilberintrusion (Meßbereich : 1 µm bis 3 nm Porendurchmesser) ermittelte spezifische Oberfläche verstanden.
Der mittlere Porendurchmesser ist in dieser Schrift als das vierfache des Verhältnisses von gemäß vorgenannter Quecksilberintrusionsmethode ermitteltem Porengesamtvolumen zu spezifischer Oberfläche O definiert.

Prinzipiell können erfindungsgemäß geeignete Multimetalloxidaktivmassen (I) in einfacher Weise dadurch hergestellt werden, daß man von geeigneten Quellen ihrer elementaren Konstituenten eine wäßrige Lösung erzeugt, diese sprühtrocknet (Austrittstemperaturen zweckmäßig 100 bis 150°C) und anschließend in einem rotierenden Behälter calciniert. Üblicherweise beträgt die Calcinationstemperatur 450 bis 1000, vorzugsweise 450 bis 700, häufig 450 bis 600 oder 550 bis 570°C.
Die Calcination kann sowohl unter Inertgas als auch unter einer oxidativen Atmosphäre wie z.B. Luft oder Gemischen aus Inertgas und Sauerstoff sowie auch unter reduzierender Atmosphäre, z.B. unter Gemischen aus Inertgas, Sauerstoff und NH₃, CO und/oder H₂, erfolgen. Dies läßt sich in einfacher Weise dadurch realisieren, daß der rotierende Behälter von einem entsprechenden Gasgemisch durchströmt wird. Als rotierender Behälter kommen z.B ein Drehrohrofen oder ein rotierender Quarzrundkolben in Betracht.

Als Quellen für die elementaren Konstituenten der Multimetalloxidaktivmassen (I) kommen solche Verbindungen in Betracht, bei denen es sich bereits um Oxide handelt und/oder solche Verbindungen, die durch Erhitzen, wenigstens in Anwesenheit von Sauerstoff, in Oxide überführbar sind.
Neben den Oxiden kommen als solche Ausgangsverbindungen vor allem Halogenide, Nitrate, Formiate, Oxalate, Citrate, Acetate, Carbonate, Aminkomplexe, Ammonium-Salze und/oder Hydroxide in Betracht (Verbindungen wie NH₄OH, (NH₄)₂CO₃, NH₄NO₃, NH₄CHO₂, CH₃COOH, NH₄CH₃CO₂ und/oder Ammoniumoxalat, die spätestens beim späteren Calcinieren zu vollständig gasförmig entweichenden Verbindungen zerfallen und/oder zersetzt werden können, können in die wäßrige Lösung zusätzlich eingearbeitet werden).Um das Lösen im wäßrigen Medium gegebenenfalls zu erleichtern, kann selbiges bei Bedarf angesäuert, mit Base versetzt und/oder auf erhöhte Temperatur gebracht werden.

Besonders geeignete Ausgangsverbindungen des Mo, V, W und Nb sind deren Oxoverbindungen (Molybdate, Vanadate, Wolframate
und Niobate) bzw. die von diesen abgeleiteten Säuren (Ammoniummolybdat, Ammoniumvanadat, Ammoniumwolframat).

Für die gasphasenkatalytische oxidative Dehydrierung von Propan zu Propen können die erfindungsgemäßen Multimetalloxidmassen (I) sowohl in Pulverform als auch zu bestimmten Katalysatorgeometrien geformt eingesetzt werden, wobei die Formgebung vor oder nach der abschließenden Calcination erfolgen kann. Beispielsweise können aus der Pulverform der Aktivmasse oder ihrer uncalcinierten Vorläufermasse durch Verdichten zur gewünschten Katalysatorgeometrie (z.B. durch Tablettieren, Extrudieren oder Strangpressen) Vollkatalysatoren hergestellt werden, wobei gegebenenfalls Hilfsmittel wie z.B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können. Geeignete Vollkatalysatorgeometrien sind z.B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm betragen kann.

Natürlich kann die Formgebung der pulverförmigen Aktivmasse oder ihrer pulverförmigen, noch nicht calcinierten, Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie er z.B. aus der DE-A 2909671 oder aus der EP-A 293859 bekannt ist. Zweckmäßigerweise kann zur Beschichtung der Trägerkörper die aufzubringende Pulvermasse befeuchtet und nach dem Aufbringen, z.B. mittels heißer Luft, wieder getrocknet werden. Die Schichtdicke der auf den Trägerkörper aufgebrachten Pulvermasse wird zweckmäßigerweise im Bereich 50 bis 500 µm, bevorzugt im Bereich 150 bis 250 µm, liegend gewählt.

Als Trägermaterialien können dabei übliche poröse oder unporöse Aluminiumoxide, Siliciumdioxid, Thoriumdioxid, Zirkondioxid, Siliciumcarbid oder Silicate wie Magnesium- oder Aluminiumsilikat verwendet werden. Die Trägerkörper können regelmäßig oder unregelmäßig geformt sein, wobei regelmäßig geformte Trägerkörper mit deutlich ausgebildeter Oberflächenrauhigkeit, z.B. Kugeln oder Hohlzylinder, bevorzugt werden.
Geeignet ist die Verwendung von im wesentlichen unporösen, oberflächenrauhen, kugelförmigen Trägern aus Steatit, deren Durchmesser 1 bis 8 mm, bevorzugt 4 bis 5 mm beträgt.

In anwendungstechnisch zweckmäßiger Weise erfolgt die Durchführung des erfindungsgemäßen Verfahrens in Rohrbündelreaktoren wie sie z.B. in der EP-A 700893 und in der EP-A 700714 beschrieben sind. In den Metallrohren (in der Regel aus Edelstahl) befindet sich der erfindungsgemäß zu verwendende Festbettkatalysator und um die Metallrohre wird ein Temperiermedium, in der Regel eine Salzschmelze, geführt. D.h., in einfachster Weise enthält jedes Reaktionsrohr eine Schüttung eines wenigstens ein Multimetalloxid (I) als Aktivmasse aufweisenden Katalysators.
Das Reaktionsgasausgangsgemisch besteht zweckmäßig aus ≥ 50 Vol.-% Propan, ≥ 15 Vol.-% O₂ und 0 bis 35 Vol.-% Inertgas. Mit Vorteil umfaßt das Reaktionsgasausgangsgemisch ≤ 30 Vol.-%, vorzugsweise ≤ 20 Vol.-% und besonders bevorzugt ≤ 10 Vol-% bzw. ≤ 5 Vol-% Inertgas. Selbstverständlich kann das Reaktionsgasausgangsgemisch auch kein Inertgas umfassen.
Unter Inertgas werden hier solche Gase verstanden, deren Umsatz beim Durchgang des Reaktionsgasausgangsgemischs durch den erfindungsgemäß zu verwendenden Festbettkatalysator ≤ 5 mol-% beträgt. Als Inertgas kommen z.B. H₂O, CO₂, CO, N₂ und/oder Edelgase in Betracht.

Weiterhin enthält das Reaktionsgasausgangsgemisch zweckmäßig ≥ 60 Vol.-%, oder ≥ 70 Vol.-%, oder ≥ 80 Vol.-% Propan. Generell liegt der Propangehalt des erfindungsgemäß einzusetzenden Reaktionsgasausgangsgemisches bei ≤ 85 Vol.-%, häufig bei ≤ 83 oder ≤ 82 oder < 81 oder < 80 Vol.-%. Der Gehalt des Reaktionsgasausgangsgemisches an molekularem Sauerstoff kann beim erfindungsgemäßen Verfahren bis zu 35 Vol.-% betragen. Mit Vorteil liegt er bei wenigstens 20 Vol.-% oder bei wenigstens 25 Vol.-%.

Erfindungsgemäß günstige Reaktionsgasausgangsgemische enthalten ≥ 65 Vol.-% und ≤ 85 Vol.-% Propan sowie ≥ 15 Vol.-% und ≤ 35 Vol.-% molekularen Sauerstoff.
Erfindungsgemäß von Vorteil ist, wenn das Molverhältnis von Propan zu molekularem Sauerstoff im Reaktionsgasausgangsgemisch ≤ 5:1, bevorzugt ≤ 4,75:1, besser ≤ 4,5:1 und besonders bevorzugt ≤ 4:1 beträgt. In der Regel wird vorgenanntes Verhältnis ≥ 1:1 bzw. ≥ 2:1 betragen.

Der Reaktionsdruck beträgt im allgemeinen ≥ 0,5 bar. Im Regelfall wird der Reaktionsdruck 100 bar nicht überschreiten, d.h. ≥ 0,5 bis 100 bar betragen. Zweckmäßig beträgt der Reaktionsdruck häufig > 1 bis 50 bzw. > 1 bis 20 bar.
Bevorzugt liegt der Reaktionsdruck bei ≥ 1,25 bzw. ≥ 1,5 oder ≥ 1,75 bzw. ≥ 2 bar.
Häufig wird dabei die Obergrenze von 10 bzw. 20 bar nicht überschritten.
Selbstverständlich kann der Reaktionsdruck auch 1 bar betragen (vorstehende Aussagen bezüglich des Reaktionsdruckes gelten für das erfindungsgemäße Verfahren ganz generell).
Ferner wird die Belastung mit Vorteil so gewählt, daß die Verweilzeit des Reaktionsgasgemisches über die Katalysatorschüttung 0,5 bis 20 sec, bevorzugt 1 bis 10 sec, besonders bevorzugt 1 bis 4 sec und häufig 3 sec beträgt.

Wird das erfindungsgemäße Verfahren kontinuierlich durchgeführt kann im Produktgemisch enthaltenes nicht umgesetztes Propan abgetrennt und in die erfindungsgemäße katalytische oxidative Dehydrierung rückgeführt werden.

Ferner können sich an das erfindungsgemäße Verfahren weitere heterogen katalysierte Oxidationsstufen anschließen, wie sie für die heterogen katalysierte Gasphasenoxidation von Propen zu Acrolein und/oder Acrylsäure bekannt sind und wie es in der älteren Anmeldung DE-A 19751046 beschrieben ist.

### Beispiele

### Beispiel 1

a) Herstellung einer Multimetalloxidmasse I
In 3,6 kg Wasser wurden bei 45°C 877,2 g Ammoniumheptamolybdat (81,5 Gew.-% MoO₃) gelöst und zu der resultierenden Lösung 2227,2 g einer wäßrigen Cobaltnitratlösung (auf die Lösung bezogen 12,5 Gew.-% Co) zugegeben. Die entstandene klare rote Lösung wurde in einem Sprühtrockner der Fa. Niro bei einer Eingangstemperatur von 330-340°C und einer Ausgangstemperatur von 110°C sprühgetrocknet (A/S Niro Atomizer transportable Minor-Anlage). 450 g des Sprühpulvers wurden innerhalb von 40 min. mit 75 ml Wasser verknetet (1-1-Kneter vom Typ Sigmaschaufel-Kneter der Fa. Werner & Pfleiderer) und in einem Umlufttrockenschrank 16 h bei 110°C getrocknet. Anschließend wurde der getrocknete Feststoff in einem von Luft durchströmten, rotierenden (15 Umdrehungen/min) Quarzrundkolben (Innenvolumen: 2 l, Luftdurchsatz: konstant 250 l/h) wie folgt calciniert (Klappof enbeheizung) :
Zunächst wurde mit einer Aufheizrate von 180°C/h von Raumtemperatur (25°C) auf 225°C aufgeheizt. Anschließend wurde die Temperatur von 225°C während 0,5 h aufrechterhalten und danach wurde mit einer Aufheizrate von 60°C/h die Calcinationstemperatur von 225°C auf 300°C erhöht. Diese Temperatur wurde anschließend während 3 h aufrechterhalten. Danach wurde mit einer Aufheizrate von 125°C/h die Calcinationstemperatur von 300 auf 550°C erhöht. Diese Temperatur wurde anschließend während 6 h aufrechterhalten.
Das so erhaltene Multimetalloxid wurde zerkleinert und als katalytisch aktive Multimetalloxidmasse (I) der Stöchiometrie Mo₁Co_{0.95}Oₓ die Kornfraktion mit einem Korngrößtdurchmesser von0,6 bis 1,2 mm durch Sieben abgetrennt. Der mittlere Durchmesser der Poren der Aktivmasse betrug 0,027 µm und die spezifische Oberfläche betrug 33,1 m²/g.
b) Oxidative katalytische Dehydrierung von Propan
Ein Reaktionsrohr (V2A Stahl; 2,5 cm Wandstärke; 8,5 mm Innendurchmesser; elektrisch beheizt) der Länge 1,4 m wurde von unten nach oben auf einem Kontaktstuhl (7 cm Länge) zunächst auf einer Länge von 23 cm mit Quarzsplitt (zahlenmittlerer Größtdurchmesser 1 bis 2 mm ) und anschließend auf einer Länge von 75 cm mit der Multimetalloxidmasse I beschickt, bevor die Beschickung auf einer Länge von 35 cm mit Quarzsplitt (zahlenmittlerer Größtdurchmesser 1 bis 2 mm) abgeschlossen wurde.
Das wie vorstehend beschickte Reaktionsrohr wurde auf seiner gesamten Länge auf 395°C aufgeheizt und dann mit 56 Nl/h einer Reaktionsgasausgangsmischung aus 80 Vol.-% Propan und 20 Vol.-% Sauerstoff von oben nach unten beschickt. Der Druck am Reaktionsrohreingang betrug 1,6 bar (abs.). Der Druckabfall längs des Reaktionsrohres betrug 0,22 bar.
Bei einfachem Durchgang wurde ein Produktgasgemisch erhalten, das nachfolgende Charakteristik aufwies:

| | |
|---|---|
| Propanumsatz | 12,4 mol-% |
| Selektivität der Propenbildung | 69 mol-% |
| Selektivität der Acroleinbildung | 2 mol-% |
| Selektivität der Acrylsäurebildung | 4 mol-% |
| Raumzeitausbeute an Propen | 3,8 mol/l Kat·h |
| Raumzeitausbeute an Wertprodukt | 4,1 mol/l Kat·h. |

### Vergleichsbeispiel

a) Herstellung einer Multimetalloxidmasse I
In 1,2 kg Wasser wurden bei 80°C 292,4 g Ammoniumheptamolybdat (81,5 Gew.-% MoO₃) gelöst und zu der resultierenden Lösung 742,4 g wäßrige Cobaltnitratlösung (auf die Lösung bezogen 12,5 Gew.-% Co) zugegeben. Die entstandene Lösung wurde unter Rühren auf dem Wasserbad bei 100°C eingedampft, bis eine pastöse Masse entstanden war. Die pastöse Masse wurde während 40 min mit 30 ml Wasser verknetet (1-l-Kneter vom Typ Sigmaschaufel-Kneter der Fa. Werner & Pfleiderer). Die resultierende Masse wurde in einem Trockenschrank 16 h bei 110°C getrocknet und anschließend in einem luftdurchströmten Muffelofen (60 l Innenvolumen, Luftdurchsatz 500 l/h) wie folgt calciniert:
Zunächst wurde mit einer Aufheizrate von 120°C/h von 25°C auf 300°C aufgeheizt.
Anschließend wurde die Temperatur von 300°C während 3 h aufrechterhalten und danach wurde mit einer Aufheizrate von 125°C/h die Calcinationstemperatur von 300 auf 550°C erhöht. Diese Temperatur wurde anschließend während 6 h aufrechterhalten. Das so erhaltene Multimetalloxid wurde zerkleinert und als katalytisch aktive Multimetalloxidmasse I der Stöchiometrie Mo₁Co_{0,95}Oₓ die Kornfraktion mit einem Korngrößtdurchmesser von 0,6 bis 1,2 mm durch Sieben abgetrennt. Der mittlere Durchmesser der Poren der Aktivmasse lag bei 0,048 µm und die spezifische Oberfläche betrug 28,4 m².
b) Oxidative katalytische Dehydrierung von Propan
Die oxidative katalytische Dehydrierung von Propan wurde wie in Beispiel 1 durchgeführt, die eingesetzte Aktivmasse war jedoch jene gemäß Vergleichsbeispiel, a).
Bei einfachem Durchgang wurde ein Produktgasgemisch erhalten, das nachfolgende Charakteristik aufwies:

| | |
|---|---|
| Propanumsatz | 8,8 mol-% |
| Selektivität der Propenbildung | 76 mol-% |
| Selektivität der Acroleinbildung | 2 mol-% |
| Selektivität der Acrylsäurebildung | 2 mol-% |
| Raumzeitausbeute an Propen | 2,9 mol/l Kat·h |
| Raumzeitausbeute an Wertprodukt | 3,1 mol/l Kat·h. |

### Beispiel 2

a) Herstellung einer Multimetalloxidmasse I
In 3,6 kg Wasser wurden bei 45°C 877,2 g Ammoniumheptamolybdat (81,5 Gew.-% MoO₃) gelöst und zu der resultierenden Lösung 2227,2 g wäßrige Cobaltnitratlösung (auf die Lösung bezogen 12,5 Gew.-% Co) zugegeben.
Die entstandene klare rote Lösung wurde in einem Sprühtrockner wie in Beispiel 1, a) sprühgetrocknet. 450 g des Sprühpulvers wurden wie in Beispiel 1, a) mit Wasser verknetet und anschließend getrocknet und calciniert. Im Unterschied zu Beispiel 1, a) wurde bei der Calcinierung abschließend die Calcinationstemperatur mit einer Aufheizrate von 133°C/h von 300°C auf 565°C erhöht und diese Calcinationstemperatur für 6 h aufrechterhalten.
Das so erhaltene Multimetalloxid wurde zerkleinert und als katalytisch aktive Multimetalloxidmasse I der Stöchiometrie Mo₁Co_{0,95}Oₓ die Kornfraktion mit einem Korngrößtdurchmesser von 0,6 bis 1,2 mm durch Sieben abgetrennt.
Der mittlere Durchmesser der Poren der Aktivmasse lag bei 0,024 µm und die spezifische Oberfläche betrug 29,2 m²/g.
b) Oxidative katalytische Dehydrierung von Propan
Wie in Beispiel 1 wurde ein Reaktionsrohr mit der Aktivmasse aus Beispiel 2 befüllt. Das befüllte Reaktionsrohr wurde auf seiner gesamten Länge auf 400°C aufgeheizt und dann mit 112 Nl/h einer Reaktionsgasausgangsmischung aus 80 Vol.-% Propan und 20 Vol.-% Sauerstoff beschickt. Der Druck am Reaktoreingang betrug 2,7 bar (abs.). Der Druckabfall längs des Reaktionsrohres betrug 0,5 bar.
Bei einfachem Durchgang wurde ein Produktgasgemisch erhalten, das nachfolgende Charakteristik aufwies :

| | |
|---|---|
| Propanumsatz | 9,4 mol-% |
| Selektivität der Propenbildung | 77 mol-% |
| Selektivität der Acroleinbildung | 2 mol-% |
| Selektivität der Acrylsäurebildung | 3 mol-% |
| Raumzeitausbeute an Propen | 6,3 mol/l Kat·h |
| Raumzeitausbeute an Wertprodukt | 6,8 mol/l Kat·h. |

## Patentansprüche

1. Multimetalloxidmassen der allgemeinen Formel I
Mₐ¹Mo_{1-b}M_{b}²Oₓ (I),
mit
M¹ = Co, Ni, Mg, Zn, Mn und/oder Cu,
M² = W, V, Te, Nb, P, Cr, Fe, Sb, Ce, Sn und/oder La,
a = 0,5 bis 1,5 ,
b = 0 bis 0,5 , sowie
x = eine Zahl, die durch die Wertigkeit und Häufigkeit der von Sauerstoff verschiedenen Elemente in (I) bestimmt wird,
mit der Maßgabe, daß
der mittlere Durchmesser der Poren der Multimetalloxidmasse (I) ≤ 0,04 µm und ≥ 0,01 µm und
die spezifische Oberfläche der Multimetalloxidmasse (I) ≥ 20 m²/g beträgt.

2. Verfahren der oxidativen katalytischen Dehydrierung von Propan zu Propen, **dadurch gekennzeichnet, daß** als Katalysator ein solcher eingesetzt wird, dessen Aktivmasse eine Multimetalloxidmasse gemäß Anspruch 1 ist.

## Claims

1. A multimetal oxide material of the formula I
Mₐ¹Mo_{1-b}M_{b}²Oₓ (I),
where
M¹ = Co, Ni, Mg, Zn, Mn and/or Cu,
M² = W, V, Te, Nb, P, Cr, Fe, Sb, Ce, Sn and/or La
a = 0.5 to 1.5,
b = 0 to 0.5, and
x = is a number which is determined by the valence and frequency of the elements in (I) other than oxygen,
with the proviso that
the mean diameter of the pores of the multimetal oxide material (I) is s 0.04 µm and ≥ 0.01 µm and
the specific surface area of the multimetal oxide material (I) is ≥ 20 m²/g.

2. A process for the oxidative catalytic dehydrogenation of propane to propene, wherein the catalyst used is one whose active material is a multimetal oxide material as claimed in claim 1.

## Revendications

1. Masses d'oxydes polymétalliques répondant à la formule générale I:
Mₐ¹Mo_{1-b}M_{b}²Oₓ (I)
où
M¹ = Co, Ni, Mg, Zn, Mn et/ou Cu,
M² = W, V, Te, Nb, P, Cr, Fe, Sb, Ce, Sn et/ou La,
a = 0,5 à 1,5,
b = 0 à 0,5, ainsi que
x = un nombre qui est déterminé par la valence et la fréquence des éléments différents de l'oxygène dans (I),
à la condition que
le diamètre moyen des pores de la masse d'oxydes polymétalliques (I) soit ≤ 0,04 µm et ≥ 0,01 µm, et que
la surface spécifique de la masse d'oxydes polymétalliques (1) soit ≥ 20 m²/g.

2. Procédé de déshydrogénation catalytique par oxydation de propane en propène, **caractérisé en ce qu'**on met en oeuvre, comme catalyseur, un catalyseur dont la masse active est une masse d'oxydes polymétalliques suivant la revendication 1.
